# EUROPEAN PATENT SPECIFICATION

(11) **EP 4 309 611 B1**
(45) Date of publication and mention of the grant of the patent: **30.09.2026**
(21) Application number: 22382698.3
(22) Date of filing: 20.07.2022
(51) Int. Cl.: A61B 34/30

(54) **PARKING ROBOT SYSTEM**
PARKROBOTERSYSTEM
SYSTÈME DE STATIONNEMENT DE ROBOT

(43) Date of publication of application: 24.01.2024
(73) Proprietor: Cyber Surgery, S.L., 20009 Donostia, Guipúzcoa (ES)
(72) Inventor: LÓPEZ DEL PUEYO, Javier, E-20009 Donostia, Guipúzcoa (ES); ESCUDERO MARTÍNEZ DE IBARRETA, Álvaro, E-20009 Donostia, Guipúzcoa (ES); GARMENDIA IARTZA, Laurentzi, E-20009 Donostia, Guipúzcoa (ES); SORRELUZ PEDROSA, Mikel, E-20009 Donostia, Guipúzcoa (ES); AMARILLO ESPITIA, Andrés, E-20009 Donostia, Guipúzcoa (ES); MENDIZABAL DONES, Ainitze, E-20009 Donostia, Guipúzcoa (ES)
(74) Representative: ABG Intellectual Property Law, S.L.

(56) References cited:
- EP-A1- 3 241 747
- WO-A1-2018/053281
- WO-A1-97/29690
- CN-A- 106 625 677
- CN-A- 109 431 608
- CN-A- 113 786 243
- CN-U- 214 073 575
- US-A1- 2021 347 060

## Description

### OBJECT OF THE INVENTION

The present invention relates to surgical assistance robot system and in particular to a system for positioning a surgical robot in surgical interventions of a patient and in turn for storing the robot in a parking position.

### BACKGROUND OF THE INVENTION

According to the prior art, robot arms show a plurality of degrees of freedom from the base to the end-effector, the end-effector located at the end of a kinematic chain comprising rigid sections connected by joints. Each joint provides one or more degrees of freedom among which are a linear movement, a rotational movement or a spherical movement.

Robot systems are classified depending on the combination of the degrees of freedom of each joint. A very important class of robot system is called *polyarticulated* and usually has a fixed base, a robot arm and an end-effector.

A second type of robot system is called *mobile.* This type of robot system has a great ability to move and is usually based on the use of carts or platforms and equipped with a rolling locomotive system. These robots follow their path by remote control or guided by information received from their environment through sensors.

These robots, for example, ensure the transport of parts from one point to another on a production line. Guided by materialized tracks through electromagnetic radiation from circuits embedded in the floor, or through photo-electrically detected bands, or they can even manage to avoid obstacles if they are endowed with a relatively high level of intelligence.

When a robot has its base fixed, the end-effector of the robot arm has a maximum reach and, for boundary points within the space reachable by the end-effector, can also only be reached with a limited set of orientation angles.

These are the limits that have to be taken into account when designing a set of actuations or movements that are repeatedly applied for example in an assembly line and, in general, in any activity that is processed by the robot system.

One of the work environments in which a robot system is becoming more and more relevant is in surgical environments where the actions that until now were carried out by a surgeon by hand are now performed by a robot system.

One of the reasons why the use of robots is acquiring this role is because a robot arm is able to act with movements that have a very high precision. This kind of precision is not possible to be achieved by a human being acting through his hands.

In order to be able to perform a surgical operation using a robot arm, it is clear that such a device must be integrated into the rest of the elements that make up an operating room by fulfilling various requirements of a very demanding nature.

Among the most relevant requirements are the following:
- The robot system must have sufficient scope to reach the surgical region in such a way that the robot arm does not enter into contact with the patient, the surgical table, the surgical area lighting system or any other element necessary for monitoring and acting on the patient.
- The base of the robotic system should be placed in a location that does not impede the presence of a surgeon and, when the operation does not require a robotic system, the robotic system should be able to be fall-back clearing the area around the surgical table.
- If possible, the robot system should be able to be stored in the same operating room while minimizing the space occupied.
- If possible, it should be possible to move the robot base.
- Finally, the robot base, while maintaining the above requirements, must be able to maintain positional stability to ensure that the end-effector is not disturbed in position, orientation or both. Likewise, the configuration of the robot system must be such that it is not exposed in storage to avoid damage or contamination from exposure.

According to a first aspect of the invention, these problems and drawbacks are solved by means of the specific configuration of a surgical assistance robot system for positioning a surgical robot. Document EP3241747 A1 describes a device for receiving a drone and associated system for distributing drones.

Document CN113786243 A describes a Miniature remote surgical robot master hand transport cart.

Document WO2018/053281 A1 describes table adapters for mounting robotic arms to a surgical table.

Document US2021/347060 A1 describes a wheeled base of a Smart logistics robotic assistant.

Document CN214073575 U describes a nested auxiliary minimally invasive surgery single-port surgical robot.

Document CN109431608 A describes an embedded auxiliary single-pore surgical robot for minimally invasive surgery department.

Document CN106625677 A describes a folding type hard disk array operation robot.

Document WO97/29690 A1 describes a method and apparatus for performing minimally invasive cardiac procedures.

### DESCRIPTION OF THE INVENTION

The present invention proposes a solution to the preceding problems by means of a system according to claim 1. The dependent claims define the preferred embodiments of the invention.

A first aspect of the invention provides a *system comprising a support structure and a robot positioner.* The support structure is intended to support the robot positioner which in turn is intended for positioning a robot. Specifically, the robot positioner will be understood as a set of elements of the surgical assistant responsible for establishing the physical link between a robot and a support structure.

Along the description, a robot will be understood as a device comprising a fixing base, a robot arm comprising a set of rigid sections connected by joints having one or more degrees of freedom and, an end-effector.

*The support structure comprises a prismatic body with a polygonal base and with vertical vertices perpendicular to the base limiting lateral faces of the prismatic body.* In this document, the term "vertex" will be understood as a line of the prismatic body where the lateral faces intersect. According to a cross sectional view of the prismatic body the lateral faces are lines intersecting at the vertex that results in a point. The vertex or, according to a cross sectional view a point, can be also considered as a reference which physically does not necessarily is identified with a physical element or part of it, e.g. when both lateral faces do not intersect at an edge but at a curved section. In this example the vertex is a fictitious line that is defined by the intersection of two lateral faces. In other words, a vertical vertex will be understood as an edge (which physically does not necessarily occur) passing through each vertex that the prismatic body comprises. In a particular embodiment, *the polygonal base is quadrangular.* In this particular embodiment, the prismatic body has four vertical vertex. This configuration of a quadrangular base allows a better use of internal space, as most of the components (controller, electrical panel, batteries, etc.) have a parallelepiped shape.

*The support structure also comprises a cavity with an opening that at least starts from a first vertical vertex and extends horizontally along a first lateral face to a second vertical vertex adjacent to the first vertical vertex*

The prismatic body of the support structure is understood as a closed rigid body comprising a cavity that is accessible by means of an opening from at least a first lateral face of the support structure. In a particular embodiment, the opening of the cavity is not only extended from the first lateral face of the support structure but extends above the support structure itself. So that the cavity is accessible at least from above the support structure and from the first lateral face. This is to be understood as meaning that the cavity according to this last example is an open cavity without the roof. In all cases, the cavity is an inner space of the prismatic body being limited by a window or opening bounded by a closed path being a boundary extended on the external surface of the body.

Specifically, the fact that the opening of the cavity extends horizontally from a first vertical vertex to a second vertical vertex should be interpreted as meaning that the start and end references are according to a vertical projection. This makes it possible to consider a vertical vertex as an extension axis of the referred vertex even if the cavity causes that the vertex does not physically exist.

In a more particular embodiment, *the opening of the cavity additionally extends horizontally along a second lateral face adjacent to the first lateral face, the second lateral face extended at least partially between the second vertical vertex and a third vertical vertex.*

Since the support structure comprises a prismatic body with a polygonal base, the support structure has as many lateral sides as vertical vertex or edges.

*The robot positioner comprises a vertical support being arranged along the first vertical vertex and comprises a first degree of freedom corresponding to a rotation with respect to a vertical axis.* The vertical support is configured to rotate with respect to a vertical axis. In a particular embodiment, the vertical axis is parallel to vertical vertex of the support structure and therefore orthogonal to the base of the polygonal base of the prismatic body.

This vertical support is coupled to the support structure although its rotational movement is not linked to the movement of the support structure in the way that this support structure also rotates, i.e., the vertical support is linked to the support structure by a rotating degree of freedom around a vertical axis coinciding with the first vertical vertex of the support structure. The rotation of the vertical support allows the robot positioner to move in an XY plane, a plane parallel to the polygonal base that in operative manner is horizontal. Hereinafter the term horizontal and vertical will refer to the base and the base is intended to be horizontal in operative manner taking the vertical direction as reference, being the direction of the action of gravity.

*The robot positioner further comprises at least a first support section extending horizontally from a first end located at the vertical support and a second end opposite to the first end.* The first support section extends from the vertical support perpendicular to this vertical support, i.e., perpendicular to the vertical axis.

*The robot positioner is configured to be coupled at the second end of the first support section to a robot, the robot comprising an end effector.* The robot positioner is adapted to support and to position a robot to which the second end of the first support section can be coupled, either directly or by means of additional elements as is the case according to various examples of realization.

*The system has at least one robot storage position wherein the first support section and the robot with the end effector are housed inside of the cavity.* This robot storage position, also referred to as the parking position, is the position in which part of the robot positioner and the robot coupled to it are stored in the cavity of the support structure. Thus, the support structure is intended to support the robot positioner and consequently the robot as well, and to store them. In this robot storage position, the vertical support of the robot positioner is rotated so that the first support section of the robot positioner is housed inside the cavity together with the robot and its end-effector.

Being housed inside the cavity will be interpreted as meaning that at least part of the elements that are housed in the cavity are inside it. This is the case, as an example, in which an arm or section is kept covering part of the opening of the cavity with part of the arm outside the opening and part inside the opening.

The system also comprises at least one robot working position, as opposed to the robot storage position, in which the robot positioner is deployed, i.e., outside the cavity, so that the robot is also outside the cavity ready to intervene according to the needs of the surgery. The system has a set up process that is the process of commissioning that starts from the robot storage position (or parking position) to the robot working position where the robot reach a ready to operate state (or position).

The system has at least one degree of freedom (which is the rotation of the vertical support of the robot positioner) that allows to position the robot closer to a patient, in the surgery area, so that the working space of the robot is sufficient to reach the areas of interest on the patient's body where the surgery is to be performed.

The present system provides the advantage to allow storing and positioning a robot depending on needs, whether the robot is to be used in a surgical operation or not. In particular, in the robot storage position the system keeps the robot and part of the robot positioner protected as they are housed inside the cavity of the support structure. This makes easier to pass through the surgery room as the robot and the robot positioner no longer take up space in the room when it is not necessary to use the robot.

The system also provides as another advantage an ease use of the system since the system presents the ability to adapt to the layout of the elements in a surgery room. For example, the present system has the ability to be used on either side of the patient during a surgery.

Furthermore, the present system provides more achievable workspace compared to prior art solutions. The working space of the robot positioner allows the robot to be positioned at many different locations so that it can reach different parts of the patient's body. In particular, since the vertical support is arranged along the first vertical vertex, the elements between said vertical support and the end-effector reach the greatest possible distance from the base and, in addition, optimize the space occupied in the cavity.

Another advantage that the system provides is that the reduction of components in the system compared to the prior art systems reduces complexity, costs, development time, weight, and increases reliability.

In turn, the support structure is dimensioned in such a way that it invades as little space as possible in the surgery room and at the same time allows the robot and part of the robot positioner to be stored.

The stiffness has a direct influence on the deviation at the tool tip. Good stiffness implies highfrequency, low- amplitude vibrations. Such vibrations can be minimized so that they have a small effect on the final error. The stiffness of the present system depends on how its mass is distributed and how its rigidity is also distributed. A large mass in the support structure and a small mass in both the robot positioner and the robot will contribute to the system having a high stiffness. There are many other factors that could contribute to loss of stiffness. These factors could be, among others, the number and type of degrees of freedom and their configuration. Another factor could be the presence of elements acting as dampers as for example wheel contact strips which are usually made of elastomeric polymers. In the present case, the vibrations are mainly caused by two types of disturbances which are the movement of the robot and the vibration transmitted by the tool supported by a tool guide arranged on the end-effector of the robot.

In a particular embodiment, *the connection between the robot positioner and the robot further comprises an intermediate support section through which the robot positioner is configured to be coupled to the robot, this intermediate support section extends from a first end connected to the second end of the first support section and a second end opposite to the first end, the connection between the intermediate support section and the first support section being the connection having a degree of freedom corresponding to a rotation about the vertical axis.* This intermediate support section is able to rotate with respect to the vertical axis in the connection between the intermediate support section and the first support section. More particularly, the intermediate support section extends horizontally from the first support section according to a direction orthogonal to the vertical axis. The resulting movement of the first support section and the intermediate support section caused by rotations with respect to the vertical axis are horizontal.

In a more particular embodiment, *the robot positioner comprises a plurality of support sections articulated between them forming a kinematic chain that extends from the first support section and the intermediate support section.* More particularly, *the plurality of support sections comprises a second support section extending from a first end connected to the second end of the first support section and a second end connected to the first end of the intermediate support section, and wherein at the robot storage position the plurality of support sections are also housed inside of the cavity.*

These embodiments provides a robot positioner consisting of a plurality of support sections that link together to form a kinematic chain from the vertical support to the intermediate support structure, and consequently also with the robot. Advantageously, this configuration of a plurality of support sections gives the robot a greater working reach by extending each of the support sections of the robot positioner.

In a particular embodiment, *the vertical support comprises an upper end having a second degree of freedom corresponding to a vertical displacement according to a direction parallel to the vertical axis displaceable at least between a first lower position and a second upper position, the first end of the first support section being located at the upper end of the vertical support; and wherein at the robot storage position the upper end of the vertical support is in the first lower position.* The first support section extends from the upper end of this vertical support so that when the vertical support vertically moves along a vertical axis the first support section is also moved in solidarity. This vertical displacement of the vertical support from the first lower position to the second upper position gives the robot positioner and consequently the robot greater reach, allowing the robot to reach even further from its fixed base, which is the system, and in particular the support structure.

On the other hand, the passage from the second upper position to the first lower position by a vertical displacement of the vertical support allows the robot and part of the robot positioner to remain at a height coinciding with the cavity of the support structure where they are housed in the robot storage position. In other words, the reach of the robot in surgery and for storage is improved.

In a particular embodiment, *the vertical support is arranged along the first vertical vertex as a projection out of the prismatic body.* The vertical support coincides with the first vertical vertex of the prismatic body and protrudes from this prismatic body as a projection. The fact that this vertical support protrudes from the prismatic body, that is, it goes beyond this prismatic body, would provide instability to the system which has been compensated giving more mass to the support structure so that the present system provides a compromise between stability, stiffness and robot reach. In this embodiment, the first vertical vertex is where the greatest weight has to be supported by the support structure since it is where the robot positioner is arranged or fixed to the support structure and, protruding from the base the location of this vertex element ensures that the center of gravity remains within the base area and therefore no overturning torque is generated.

In a particular embodiment, *the support structure further comprises displacement means arranged in the base of the prismatic body.* The displacement means are configured to move the support structure as required, and this advantageously allows the system to be moved through the surgery room. In this sense, in the robot storage position, the system can be positioned in the desired place so as not to disturb while it is not required to be used. In a robot working position the displacement means allows the system to be moved to improve the robot's reach. Therefore, the displacement means advantageously allows the system to be moved as needed, regardless of their position.

These means of displacement also allow the robot system to be placed in an unobtrusive part of the operating room when it needs to be stored.

Likewise, during an operation on an operative region on a patient, the displacement means allow the position of the support structure to be adjusted to optimize its position so that it does not interfere with specific space requirements. In addition, one of the main purposes of the robot positioner is to provide sufficient reachability during operation, i.e., the robot positioner is dimensioned to be able to position the robot along an entire column.

In a particular embodiment, *the support structure comprises a polygonal base support on which the prismatic body is supported in a fix way, and wherein according to a plan view orthogonal to the vertical axis, the polygonal base support goes beyond the prismatic body and partially surrounds the base of the prismatic body (2) so that this base of the prismatic body is embedded in the base support.* This polygonal base support provides supports to the support structure and consequently to the whole system so that the stability of the whole system is increased. Furthermore, the fact that this polygonal base support is protruding from the prismatic body according to a plan view orthogonal to the vertical axis means that any impact on the system is directly on this base support and not on the prismatic body. Therefore, this base support will be also understood as a protection of the system.

Additionally, since the polygonal base support is protruding from the prismatic body the stability area is also enlarged increasing the distance of the center of gravity to any point of the perimeter increasing the stability of the whole system.

In a more particular embodiment, *the polygonal base support comprises a protruding portion projecting from each vertex of the polygonal base support out of the same, and the displacement means comprises a wheel arranged at each protruding portion.* Providing the displacement means at the protruding portions that project from each vertex advantageously improves the system stability since the area determining that the system is stable when the center of gravity is located within such area is enlarged with this configuration.

When the wheels are free to change direction the actual support to allow the supporting structure to move in any direction is not always in the same place, hence in these cases it is necessary to extend the stability area since there is a perimeter zone that generates uncertainty depending on the position and orientation in which the wheels are.

In a particular embodiment, *the system has at least a first robot working position wherein the vertical support is rotated around the vertical axis relative to its position at the robot storage position of the system so that the kinematic chain formed at least by the first support section and the robot with the end effector moves away from the inside of the cavity and remains outside this cavity.*

This first working position defines a first deployment position for the system in which the robot and the robot positioner move from being housed in the cavity to going out of the cavity.

In a more particular embodiment, *the system has at least a second robot working position wherein the vertical support is rotated around the vertical axis and relative to its position at the robot storage position of the system and displaced from the first lower position to the second upper position so that the kinematic chain formed at least by the first support section and the robot with the end effector moves away from the support structure.*

The second working position defines a deployment position of the system that goes beyond the previous (first), where the robot and the robot positioner are not only outside the cavity but also move away from the support structure.

In a more particular embodiment, *the intermediate support section of the* robot *positioner is connected to a first section of the robot so that the first section of the robot constitute a single support section with the intermediate support section for the* robot *positioner.* In this way, the first section of the robot and the intermediate support section work as a single support section in the kinematic chain of the robot positioner.

The present system presents at least three specific configurations which provide a lineal positioner, a fixed positioner and angular positioner. Depending on each of these positioner configurations, part of the positioner itself and the robot are housed in the cavity in a specific way in the robot storage position of the system. These robot positioner configurations are explained in detailed below.

In a particular embodiment, *at the robot storage position the first support section is oriented towards the second lateral face.* This embodiment corresponds both to the lineal and fixed positioner, wherein the first support section of the robot positioner is oriented towards the second lateral face of the support structure, that is, is oriented towards the opening of the cavity at the second lateral face of the support structure.

In another particular embodiment, *at the robot storage position the first support section is oriented towards a third lateral face adjacent to the second lateral face.* This embodiment corresponds to the angular positioner wherein the first support section is not oriented towards the inside of the cavity and not towards the opening of the cavity.

In a particular embodiment, *the system further comprises a robot coupled to the robot positioner at the second end of the first support section, wherein at the robot storage position the robot is housed inside the cavity.*

### DESCRIPTION OF THE DRAWINGS

These and other features and advantages of the invention will be more clearly understood based on the following detailed description of a preferred embodiment given only by way of illustrative and non-limiting example in reference to the attached drawings.
Figure 1 schematically shows a perspective view of a system according to an embodiment of the present invention.
Figure 2 schematically shows another perspective view of a system according to an embodiment of the present invention.
Figure 3 schematically shows another perspective view of a system according to an embodiment of the present invention.
Figure 4 schematically shows a top view of the system shown on figure 1.
Figure 5 schematically shows a bottom view of a shown on figure 1.
Figure 6 schematically shows a sequence of movements of a robot positioner of a system according to an embodiment of the present invention.
Figure 7 schematically shows another sequence of movements of a robot positioner of a system according to an embodiment of the present invention.
Figure 8 schematically shows another sequence of movements of a robot positioner of a system according to an embodiment of the present invention.

### DETAILED DESCRIPTION OF THE INVENTION

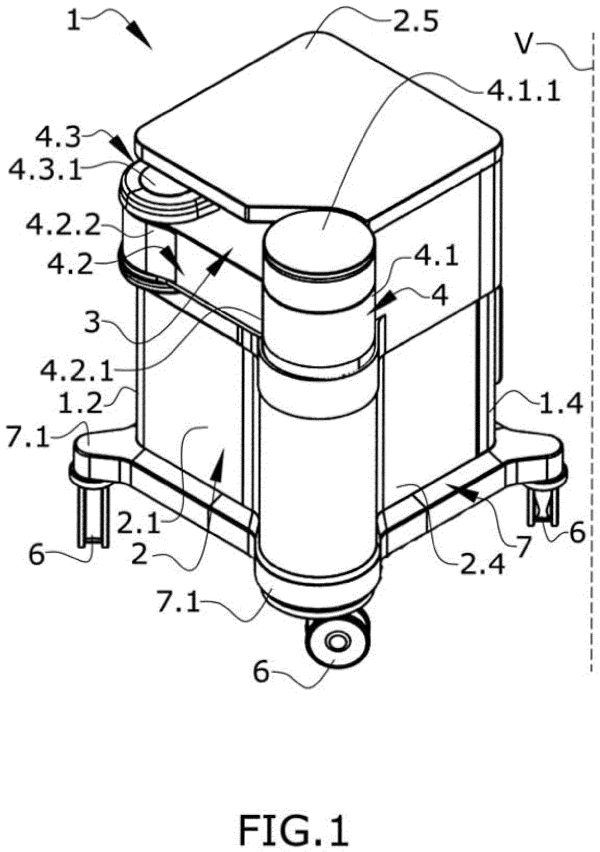

Figures 1 to 5 show different views of a system according to an embodiment of the present invention. Figures 1 to 3 show in particular a perspective view of the system in a different position in each figure. The system according to this embodiment comprises a support structure (1) and a robot positioner (4) attached to the support structure (1), specifically attached to one vertex of the support structure (1).

Along the description, it will be understood that two vertex are adjacent if the is not any intermediate vertex between said two adjacent vertex. In the same way, it will be understood that two lateral faces are adjacent if there is not any intermediate face between said two adjacent vertices. These criteria correspond to vertices and lateral faces where between two lateral faces there is a vertex and between two vertices there is a lateral face.

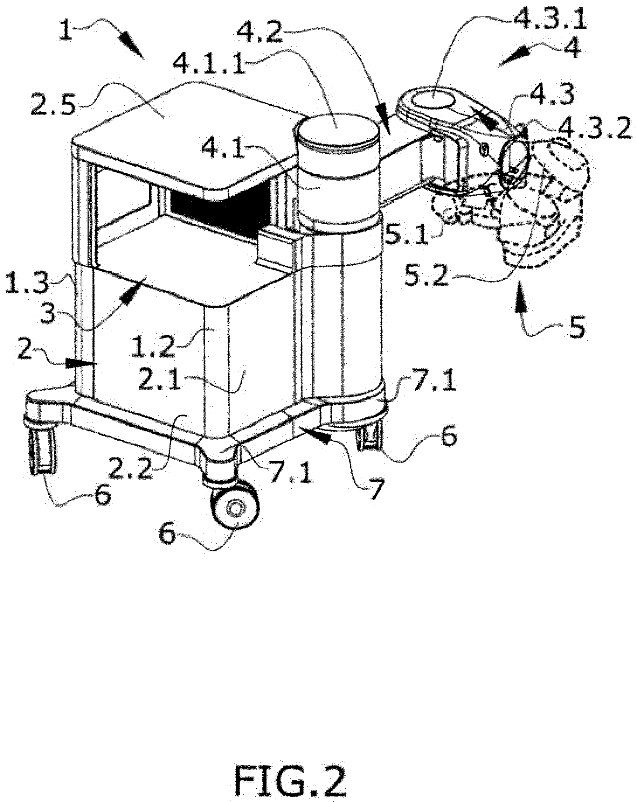

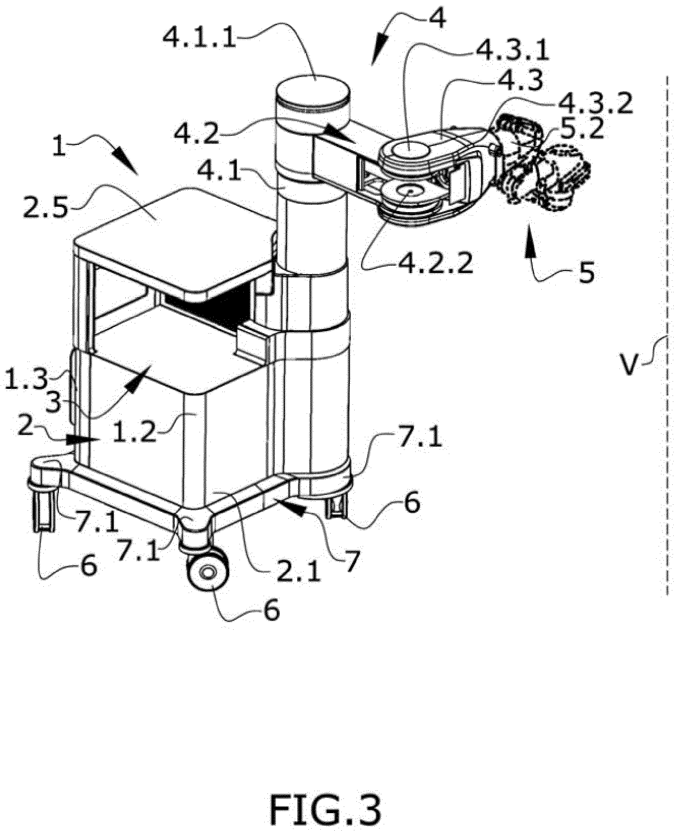

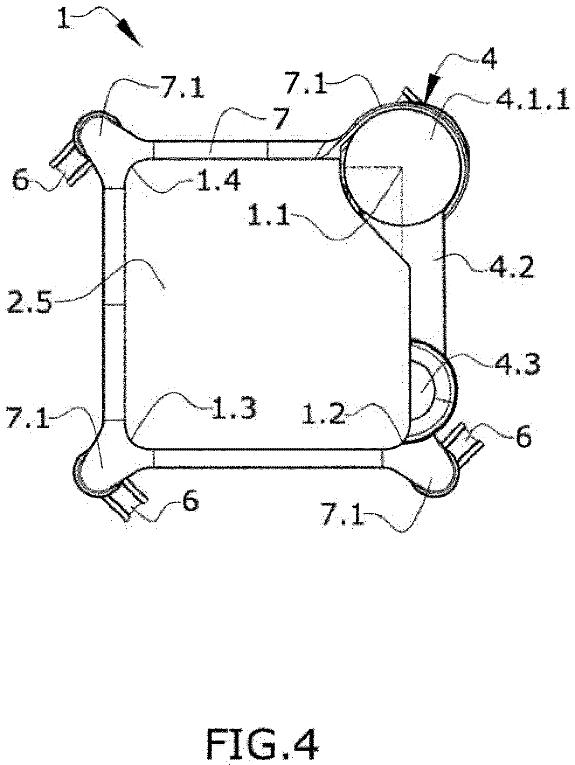

The support structure (1) comprises a prismatic body (2) having a polygonal base that is quadrangular and four vertical vertices (1.1, 1.2, 1.3, 1.4) perpendicular to the quadrangular base. In addition, the prismatic body (2) also has four lateral faces (2.1, 2.2, 2.3, 2.4) being arranged or limited by the mentioned vertical vertices (1.1, 1.2, 1.3, 1.4). In particular, the prismatic body (2) comprises a first lateral face (2.1) arranged between a first vertical vertex (1.1) and a second vertical vertex (1.2) adjacent to the first vertical vertex (1.1); a second lateral face (2.2), adjacent to the first lateral face (2.1), arranged between the second vertical vertex (1.2) and a third vertical vertex (1.3) adjacent to the second vertical vertex (1.2); a third lateral face (2.3), adjacent to the second lateral face (2.2), arranged between the third vertical vertex (1.3) and a fourth vertical vertex (1.4) adjacent to the third vertical vertex (1.3); and a fourth lateral face (2.4), adjacent to the third lateral face (2.3), arranged between the fourth vertical vertex (1.4) and the first vertical vertex (1.1) adjacent to the fourth vertical vertex (1.4). In particular, figure 1 shows only the first (2.1) and fourth (2.4) lateral faces, figures 2 and 3 shows the first (2.1) and the second (2.2) lateral faces. However, figure 4 shows all the lateral faces (2.1, 2.2, 2.3, 2.4) of the prismatic body (2) in a plan view.

In this particular embodiment, the lateral faces (2.1, 2.2, 2.3, 2.4) are contained in a plane that comprises a vertical direction parallel to a vertical axis V so that the support structure (1) is extended according to this vertical axis V.

According to these figures 1-3, the vertical vertices (1.1, 1.2, 1.3, 1.4) are understood as the edges of the prismatic body (2) where the lateral faces (2.1, 2.2, 2.3, 2.4) meet between two adjacent faces although physically this does not happen and the vertical vertices are considered in this case as a fictitious axis or edge of reference since the physical edges are rounded. In particular, the vertical vertices (1.1, 1.2, 1.3, 1.4) are parallel to the vertical axis V.

The support structure (1) further comprises a cavity (3) with an opening which allows access to the inside of the cavity (3). This opening extends from the first vertical vertex (1.1) horizontally along the first lateral face (2.1) to the second vertical vertex (1.2) and additionally from the second vertical vertex (2.1) horizontally along the second lateral face (2.2) to the third vertical vertex (1.3). Figures 2 and 3 show in detail the extent to which this cavity (3) has in the support structure (1). Specifically, these figures 2 and 3 show that the opening for the cavity (3) is limited perimetrically by a portion of the vertical support (4.1) and the third vertical vertex (1.3) of the prismatic body (2) together with a little portion of the second lateral face (2.2). In this sense, the cavity (3) is bounded on the sides by the third (2.3) and fourth (2.4) lateral sides of the prismatic body (2), on the underside by the prismatic body (2) itself and on the top by an upper surface (2.5) that may be used as a resting surface for instance for supporting a monitor for visualizing parameters of the surgical operation.

The robot positioner (4) comprises a vertical support (4.1) arranged along the first vertical vertex (1.1) of the prismatic body (2) from the base of this prismatic body (2) and extended according to the vertical axis V. This vertical support (4.1) has a first degree of freedom that corresponds to the rotation of such vertical support (4.1) with respect to the vertical axis V, that is, it is understood that the vertical support (4.1) rotates on itself.

The robot positioner (4) also comprises a first support section (4.2) that extends horizontally departing from the vertical support (4.1), in particular, the first support section (4.2) comprises a first end (4.2.1) located at the vertical support (4.1) and a second end (4.2.2) that is opposite to the first end (4.2.1). According to this embodiment, this first support section (4.2) is arranged perpendicular to the vertical axis V.

The present system also comprises an intermediate support section (4.3) that extends departing from the first support section (4.2). The intermediate support section (4.3) has a first end (4.3.1) connected to the second end (4.2.2) of the first support section (4.2) and a second end (4.3.2) that is opposite to the first end (4.3.1). This intermediate support section (4.3), according to this example, is provided for connecting the robot positioner (4) with a robot (5). The connection between the intermediate support section (4.3) and the first support section (4.2) has a degree of freedom that corresponds to the rotation of the intermediate support section (4.3) around the vertical axis V at the first end (4.3.1) of the intermediate support section (4.3).

The particular robot (5) to which the robot positioner (4) is coupled comprises an end-effector (5.1) as it can be shown on figures 2 and 3.

Figures 2 and 3 show the intermediate support section (4.3) connected at its second end (4.3.2) to a first section (5.2) of the robot (5) so that this first section (5.2) constitute a single support section with the intermediate support section (4.3) of the robot positioner (4). This is understood as the first section (5.2) of the robot (5) is also configured to rotate around the vertical axis V as a consequence of the degree of freedom of the intermediate support section (4.3).

Regarding the vertical support (4.1), this comprises an upper end (4.1.1) opposite to the base of the prismatic body (2) and having a second degree of freedom that corresponds to a vertical displacement of the upper end (4.1.1) according to a direction that is parallel to the vertical axis V. Specifically, the upper end (4.1.1) is displaceable between a first lower position (shown on figures 1 and 2) and a second upper position (shown on figure 3). Additionally, the first end (4.2.1) of the first support section (4.2) is located at the upper end (4.1.1) of the vertical support (4.1).

As it can be observed in figures 1-5, the vertical support (4.1) is arranged along the first vertical vertex (1.1) as a projection out of the prismatic body (2). That is, part of the vertical support (4.1) protrudes beyond the prismatic body (2) of the support structure (1).

The support structure (1) according to this embodiment further comprises a polygonal base support (7) on which the prismatic body (2) is supported without being able to move. This polygonal base support (7) goes beyond the prismatic body (2) and partially surrounds the base of the prismatic body (2). As it can be observed in figures 1-3, the base of the prismatic body (2) is embedded or housed in the polygonal base support (7).

The polygonal support base (7) comprises protruding portions (7.1) that protrude each one beyond this support base (7) from each vertex of the polygonal base support (7). In addition, the support structure (1) comprises displacement means (6) for displacing the system through the surgery room. According to this embodiment, the displacement means (6) are a plurality of wheels wherein each wheel (6) is located at each protruding portion (7.1), in a low position below of the polygonal base support (7).

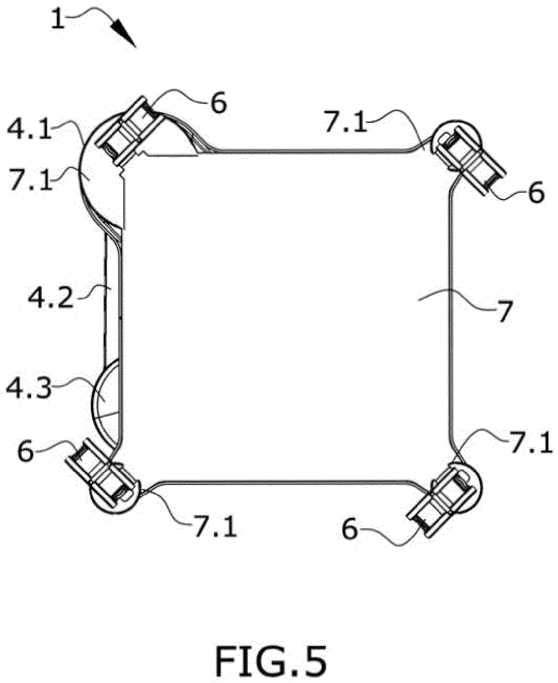

The present system has a robot storage position wherein the first support section (4.2) of the robot positioner (4) and the robot (5) with its end-effector (5.1) are housed inside the cavity (3) of the support structure (1). Figure 1 shows the system in the robot storage position, that is, the system with the robot stored in the cavity (3) of the support structure (1). According to this embodiment, part of the first support section (4.2) of the robot positioner (4) is housed inside the cavity (3) and other part of the same stay outside the cavity (3). This arrangement is compatible with the first support section (4.2) being housed in the cavity (3) since at least a portion of this first support section (4.2) is located inside the cavity (3). Figures 4 and 5 that corresponds to an top view and bottom view respectively of the system of figure 1 show how part of the first support section (4.2) protrudes beyond the support structure (1). At this robot storage position the vertical support (4.1), and particularly its upper end (4.1.1), is placed at the first lower position as shown on figure 1.

The system also has robot working positions where the first support section (4.2) of the robot positioner (4) and the robot (5) are out of the cavity (3). Particularly, figures 2 and 3 show two robot working positions.

Figure 2 shows a first robot working position where the vertical support (4.1) of the robot positioner (4) has been rotated around the vertical axis (V) compared to the robot storage position (shown on figure 1) so that the kinematic chain formed from the first support section (4.2) to the end-effector (5.1) of the robot (5) moves away from the inside of the cavity (3) remaining the robot (5) away from the support structure (1). The passage of the system from figure 1 to figure 2 corresponds to a first movement of the robot positioner (4) and consequently of the robot (5) fixed to the positioner (4).

Figure 3 shows a second robot working position where the upper end (4.1.1) of the vertical support (4.1) is moved from the first lower position (shown on figure 2) to the second upper position so that the kinematic chain formed from the first support section (4.2) to the end-effector (5.1) of the robot (5) moves further away from the support structure (1) compared to the position in figure 2 keeping the robot (5) at a higher position. The passage of the system from figure 2 to figure 3 corresponds to a second movement of the positioner and consequently of the robot (5). This figure 3 also shows how the intermediate support section (4.3) has been rotated around the vertical axis (V) at the second end (4.2.2) of the first support section (4.2), thus moving the robot (5) further away from the support structure (1) to give the robot (5) more reach.

The vertical support (4.1) of the robot positioner (4) can still be rotated around the vertical axis (V) in comparison to figure 3 in order to position the robot (5) at the required place; and the same occurs for the intermediate support section (4.3) which can still be rotated around the vertical axis (V) at its first end (4.3.1) compared to the position in figure 3.

In another embodiment (not shown in figures), the robot robot positioner (4) comprises other support sections further to the first support section (4.2), these support sections being articulated between them forming a kinematic chain that extends between the first support section (4.2) and the intermediate support section (4.3). According to a further embodiment, in the kinematic chain any link element between the first support section (4.2) and the intermediate support section (4.3) is connected in such a way that a single degree of freedom corresponds to a rotation according to a vertical axis.

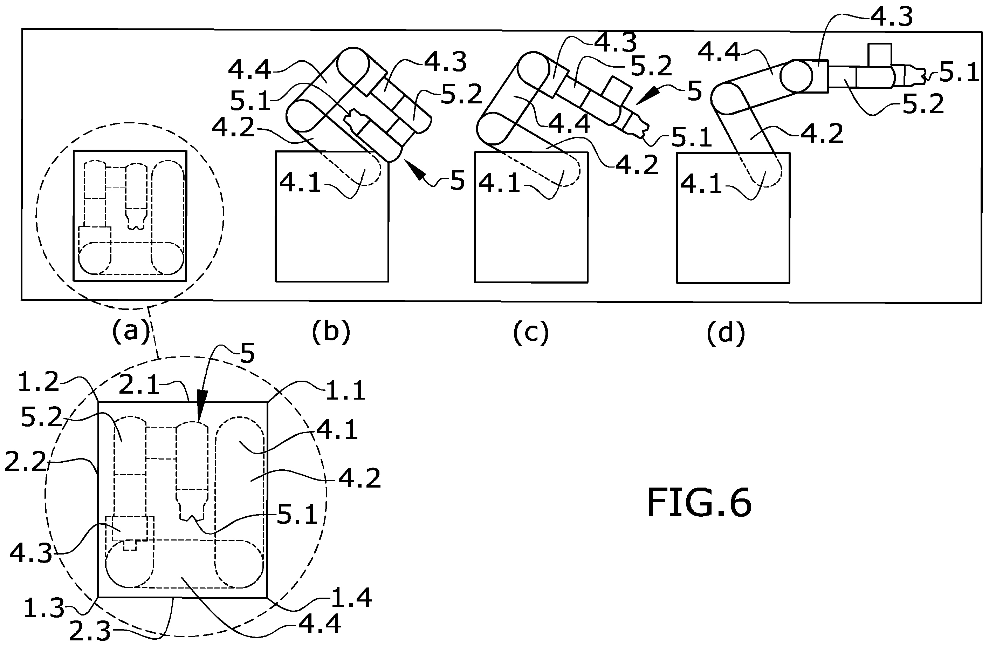

The robot positioner (4) may show at least three configurations named angular positioner, lineal positioner and fixed positioner, these positioner configurations are respectively shown in figures 6 to 8. These figures show a top view of the system according to a sequence of movements of the robot positioner (4). The system shown in these figures corresponds mainly to the system already explained above for figures 1 to 5.

Figures 6 shows a sequence of movement, from position (a) to position (d), of an angular positioner in a system according to an embodiment of the present invention. The robot positioner (4) shown on this figure 6 comprises a second support section (4.4) arranged between the first support section (4.2) and the intermediate support section (4.3). This second support section (4.4) is horizontally arranged from the second end (4.2.2) of the first support section (4.2) to the first end (4.3.1) of the intermediate support section (4.3). Specifically, the second support section (4.4) is perpendicular to the vertical axis (V) and the first support section (4.2).

Position (a) in figure 6 shows a robot storage position where the complete robot positioner (4) together with the robot (5) are housed inside the cavity (3) of the support structure. Specifically, the first support section (4.2) is arranged substantially parallel to the fourth lateral face (2.4) and closer to this fourth lateral face (2.4) and oriented towards the third lateral face (2.3). The second support section (4.4) is arranged substantially parallel to the third lateral face (2.3) and closer to this third lateral face (2.3) and oriented towards the opening of the cavity (3) above the second lateral face (2.2). The intermediate support section (4.3) together with the first section (5.2) of the robot (5) is arranges substantially parallel to the second lateral face (2.2) and closer to the opening of the cavity (3) above this second lateral face (2.2) and also oriented towards the first lateral side (2.1) or the opening of the cavity (3) above the first lateral face (2.1). The rest of the robot (5) with its end-effector (5.1) are arranged inside the cavity (3) and partially surrounded by the first (4.2) and second (4.4) support sections and the intermediate support section (4.3).

Positions (b) to (d) in figure 6 show a sequence of robot positioner (4) and robot (5) deployment movements in order to provide the robot with a large range, in particular, these positions (b) to (d) correspond to different robot working positions. Position (b) shows the robot positioner (4) practically outside the cavity (3) even with the robot (5) partially surrounded by the first (4.2) and second (4.4) support sections and the intermediate support section (4.3). Position (c) shows the robot positioner (4) also outside the cavity (3) and the robot (5) being deployed and away from the first support section (4.2) of the robot positioner (4). Position (d) shows the robot (5) even further away than in the previous position (c). To move from position (a) to position (b) the vertical support (4.1) is rotated around the vertical axis (V) more than 90º so that the support sections (4.2, 4.3, 4.4) and the robot (5) go outside the cavity (3). To move from position (b) to position (c) the robot (5) has been rotated to achieve a deployed position. To move from position (c) to position (d) the vertical support (4.1) is further rotated around the vertical axis (V) to move further away the support sections (4.2, 4.3, 4.4) and the robot (5) from the support structure (1); and also the intermediate support section (4.3) is rotated around the vertical axis (V) with respect to the second support sections (4.4).

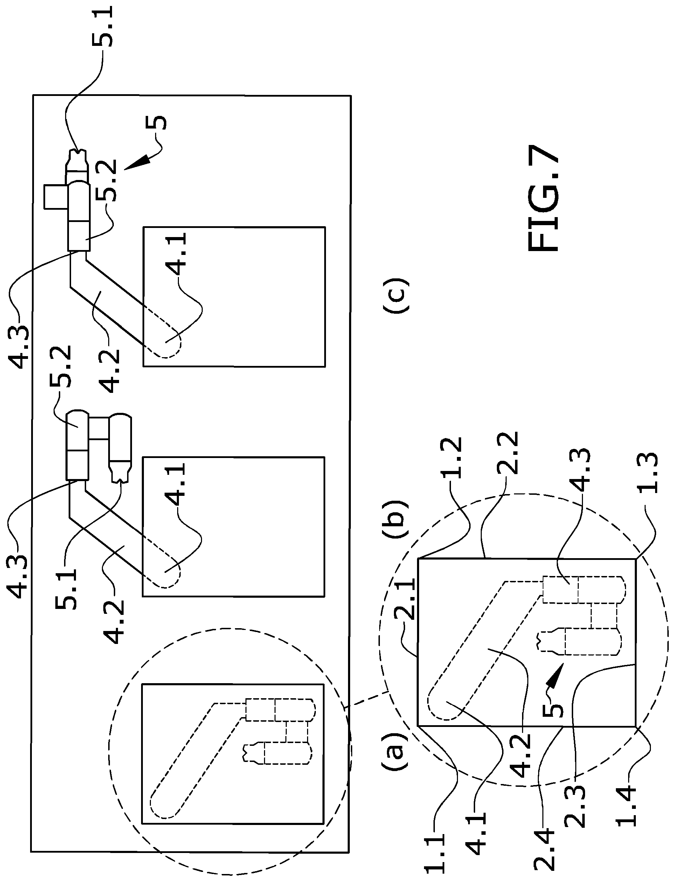

Figure 7 shows a sequence of movements, from position (a) to position (c), of a lineal positioner in a system according to an embodiment of the present invention.

Position (a) in figure 7 shows a robot storage position where the complete robot positioner (4) together with the robot (5) are housed inside the cavity (3) of the support structure (1). Specifically, the first support section (4.2) is arranged inside the cavity (3) from the vertical support (4.1) until close to the opening of the cavity (3) above the second lateral side (2.2), approximately halfway between the second vertical vertex (1.2) and the third vertical vertex (1.3). The intermediate support section (4.3) is arranged substantially parallel to the second lateral face (2.2) and closer to the opening of the cavity above the second lateral face (2.2) and oriented towards the third lateral face (2.3). The robot (5) and its end-effector (5.1) are arranged inside the cavity (3) between the third lateral face (2.3), the fourth lateral face (2.4), the intermediate support section (4.3) and the first support section (4.2).

Positions (b) to (c) in figure 7 show a sequence of robot positioner (4) and robot (5) deployment movements in order to provide the robot with range, in particular, these positions (b) to (c) correspond to different robot working positions. Position (b) shows the robot positioner (4) practically outside the cavity (3). Position (c) shows the positioner (4) also outside the cavity (3) and the robot (5) being completely deployed and away from the first support section (4.2) of the robot positioner (4). To move from position (a) to position (b) the vertical support (4.1) is rotated around the vertical axis (V) so that the support sections (4.2, 4.3) and the robot (5) go outside the cavity (3). To move from position (b) to position (c) the robot (5) has been rotated to achieve a deployed position.

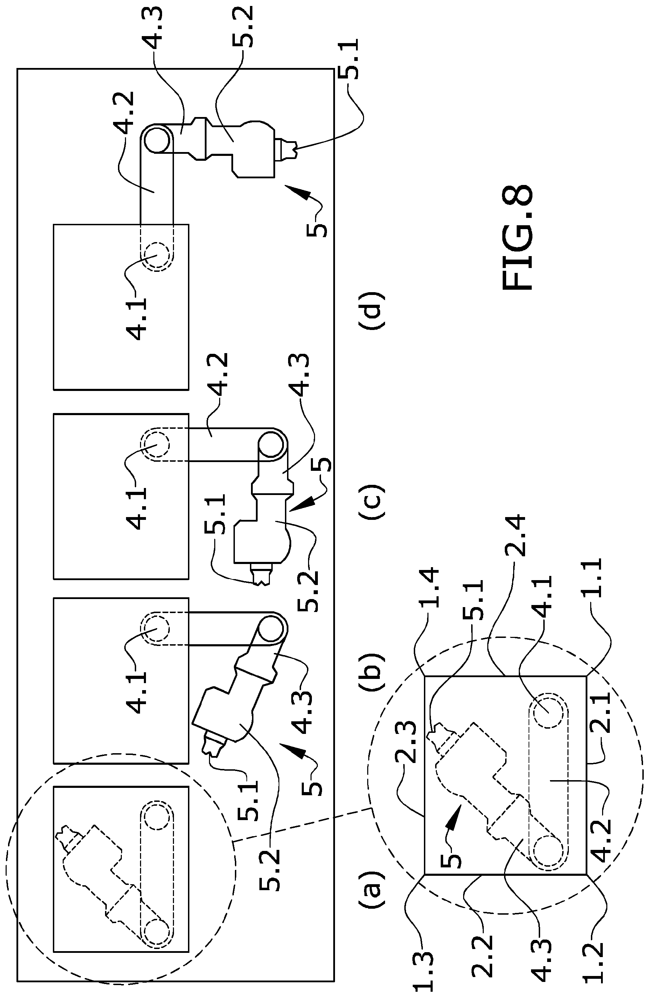

Figures 8 shows a sequence of movements, from position (a) to position (d), of a fixed positioner in a system according to an embodiment of the present invention.

Position (a) in figure 8 shows a robot storage position where the complete robot positioner (4) together with the robot (5) are housed inside the cavity (3) of the support structure (1). Specifically, the first support section (4.2) is arranged substantially parallel to the first lateral face (2.1), closer to the opening of the cavity (3) above this first lateral face (2.1) and oriented towards the opening above the second lateral face (2.2). The intermediate support section (4.3) together with the robot (5) and its end-effector (5.1) are crossed in the inside of the cavity (3) from the second end (4.2.2) of the first support section (4.2) towards approximately the halfway of the third lateral side (2.3). In this way, the robot (5) and its end-effector (5.1) are surrounded by the first support section (4.2), the fourth lateral side (2.4), the third lateral side (2.3) and the opening above the second lateral side (2.2). According to robot storage position shown on (a), the intermediate support (4.3) followed by the robot (5) and its end-effector (5.1) forms an angle with respect to the first support structure (4.2) on the inside of the cavity (3) of less than 90º.

Positions (b) to (d) in figure 6 show a sequence of robot positioner (4) and robot (5) deployment movements in order to provide the robot with range, in particular, these positions (b) to (d) correspond to different robot working positions. Position (b) shows the robot positioner (4) practically outside the cavity (3) even with the robot (5) forming an angle less than 90º with respect to the first support section (4.2). Position (c) shows the robot positioner (4) also outside the cavity (3) and the robot (5) being deployed and away from the first support section (4.2) of the robot positioner (4), specifically forming 90º with respect to the first support section (4.2). Position (d) shows the robot (5) even further away than in the previous position (c). To move from position (a) to position (b) the vertical support (4.1) is rotated around the vertical axis (V) at least 90º so that the support sections (4.2, 4.3) and the robot (5) go outside the cavity (3). To move from position (b) to position (c) the intermediate support section (4.3) is rotated until this intermediate support section (4.3) forms 90º with respect to the first support section (4.2). To move from position (c) to position (d) the vertical support (4.1) is further rotated around the vertical axis (V) to move further away the robot (5) from the support structure (1).

## Claims

1. A surgical assistance robot system for positioning a surgical robot (5), the system comprising a support structure (1) and a robot positioner (4), wherein:
the support structure (1) is configured to support the robot positioner (4) and comprises:
- a prismatic body (2) with a polygonal base and with vertical vertices (1.1, 1.2, 1.3, 1.4) perpendicular to the base limiting lateral faces (2.1, 2.2, 2.3, 2.4) of the prismatic body (2), and
- a cavity (3) with an opening that at least starts from a first vertical vertex (1.1) and extends horizontally along a first lateral face (2.1) to a second vertical vertex (1.2) adjacent to the first vertical vertex (1.1)
the robot positioner (4) is configured to position the robot (5) and comprises:
- a vertical support (4.1) being arranged along the first vertical vertex (1.1) and comprises a first degree of freedom corresponding to a rotation with respect to a vertical axis (V) coinciding with the first vertical vertex (1.1) of the support structure (1); and
- at least a first support section (4.2) extending horizontally from a first end (4.2.1) located at the vertical support (4.1) and a second end (4.2.2) opposite to the first end (4.2.1);
wherein
the robot positioner (4) is configured to be coupled at the second end (4.2.2) of the first support section (4.2) to the robot (5), the robot (5) comprising an end effector (5.1); and
the system has at least one robot storage position wherein the first support section (4.2) and the robot with the end effector (5.1) are housed inside of the cavity (3).

2. A system according to claim 1, wherein the connection between the robot positioner (4) and the robot (5) further comprises an intermediate support section (4.3) through which the robot positioner (4) is configured to be coupled to the robot (5), this intermediate support section (4.3) extends from a first end (4.3.1) connected to the second end (4.2.2) of the first support section (4.2) and a second end (4.3.2) opposite to the first end (4.3.1), the connection between the intermediate support section (4.3) and the first support section (4.2) being the connection having a degree of freedom corresponding to a rotation about the vertical axis (V).

3. A system according to claim 2, wherein the robot positioner (4) comprises a plurality of support sections articulated between them forming a kinematic chain that extends from the first support section (4.2) and the intermediate support section (4.3).

4. A system according to claim 3, wherein the plurality of support sections comprises a second support section (4.4) extending from a first end (4.4.1) connected to the second end (4.2.2) of the first support section (4.2) and a second end (4.4.2) connected to the first end (4.3.1) of the intermediate support section (4.3), and wherein at the robot storage position the plurality of support sections are also housed inside of the cavity (3).

5. A system according to any one of previous claims, wherein the vertical support (4.1) comprises an upper end (4.1.1) having a second degree of freedom corresponding to a vertical displacement according to a direction parallel to the vertical axis (V) displaceable at least between a first lower position and a second upper position, the first end (4.2.1) of the first support section (4.2) being located at the upper end (4.1.1) of the vertical support (4.1); and wherein at the robot storage position the upper end (4.1.1) of the vertical support (4.1) is in the first lower position.

6. A system according to any one of the previous claims, wherein the polygonal base is quadrangular.

7. A system according to any one of the previous claims, wherein the vertical support (4.1) is arranged along the first vertical vertex (1.1) as a projection out of the prismatic body (2).

8. A system according to any one of previous claims, wherein the support structure (1) further comprises displacement means (6) arranged in the base of the prismatic body (2).

9. A system according to any one of the previous claims, wherein the support structure (1) comprises a polygonal base support (7) on which the prismatic body (2) is supported in a fix way, and wherein according to a plan view orthogonal to the vertical axis (V), the polygonal base support (7) goes beyond the prismatic body (2) and partially surrounds the base of the prismatic body (2) so that this base of the prismatic body (2) is embedded in the base support (7).

10. A system according to claim 9, wherein the polygonal base support (7) comprises a protruding portion (7.1) projecting from each vertex of the polygonal base support (7) out of the same, and the displacement means (6) comprises a wheel arranged at each protruding portion (7.1).

11. A system according to any one of previous claims has at least a first robot working position wherein the vertical support (4.1) is rotated around the vertical axis (V) relative to its position at the robot storage position of the system so that the kinematic chain formed at least by the first support section (4.2) and the robot (5) with the end effector (5.1) moves away from the inside of the cavity (3) and remains outside this cavity (3).

12. A system according to claim 5 and any one of previous claims has at least a second robot working position wherein the vertical support (4.1) is rotated around the vertical axis (V) and relative to its position at the robot storage position of the system and displaced from the first lower position to the second upper position so that the kinematic chain formed at least by the first support section (4.2) and the robot (5) with the end effector (5.1) moves away from the support structure (1).

13. A system according to claim 2 and any one of previous claims, wherein the intermediate support section (4.3) of the positioner (4) is connected to a first section (5.2) of the robot (5) so that the first section (5.2) of the robot (5) constitute a single support section with the intermediate support section (4.3) for the positioner (4).

14. A system according to any one of previous claims, wherein the opening of the cavity (3) additionally extends horizontally along a second lateral face (2.2) adjacent to the first lateral face (2.1), the second lateral face (2.2) extended at least partially between the second vertical vertex (1.2) and a third vertical vertex (1.3).

15. A system according to any one of previous claims, wherein at the robot storage position the first support section (4.2) is oriented towards a second lateral face (2.2).

16. A system according to any one of claims 1-14, wherein at the robot storage position the first support section (4.2) is oriented towards a third lateral face (2.3) adjacent to a second lateral face (2.2).

17. A system according to any one of previous claims, further comprising a robot (5) coupled to the robot positioner (4) at the second end (4.2.2) of the first support section (4.2), wherein at the robot storage position the robot (5) is housed inside of the cavity (3).

## Patentansprüche

1. Ein chirurgisches Hilfsrobotersystem zum Positionieren eines chirurgischen Roboters (5), wobei das System eine Haltestruktur (1) und einen Roboterpositionierer (4) umfasst, wobei:
die Haltestruktur (1) konfiguriert ist, um den Roboterpositionierer (4) zu halten, und Folgendes umfasst:
- einen prismatischen Körper (2) mit einer polygonalen Basis und mit vertikalen Eckpunkten (1.1, 1.2, 1.3, 1.4) senkrecht zu den die Basis begrenzenden Seitenflächen (2.1, 2.2, 2.3, 2.4) des prismatischen Körpers (2), und
- einen Hohlraum (3) mit einer Öffnung, die mindestens von einem ersten vertikalen Eckpunkt (1.1) ausgeht und sich horizontal entlang einer ersten Seitenfläche (2.1) zu einem zweiten vertikalen Eckpunkt (1.2) neben dem ersten vertikalen Eckpunkt (1.1) erstreckt,
der Roboterpositionierer (4) konfiguriert ist, um den Roboter (5) zu positionieren, und Folgendes umfasst:
- einen vertikalen Halter (4.1), der entlang des ersten vertikalen Eckpunkts (1.1) angeordnet ist und einen ersten Freiheitsgrad umfasst, der einer Drehung in Bezug auf eine vertikale Achse (V) entspricht, die mit dem ersten vertikalen Eckpunkt (1.1) der Haltestruktur (1) zusammenfällt; und
- mindestens einen ersten Halteabschnitt (4.2), der sich horizontal von einem ersten Ende (4.2.1), das sich an dem vertikalen Halter (4.1) befindet, und einem zweiten Ende (4.2.2) gegenüber dem ersten Ende (4.2.1) erstreckt;
wobei
der Roboterpositionierer (4) konfiguriert ist, um am zweiten Ende (4.2.2) des ersten Halteabschnitts (4.2) mit dem Roboter (5) gekoppelt zu werden, wobei der Roboter (5) einen Endeffektor (5.1) umfasst; und
das System mindestens eine Roboterlagerposition aufweist, in der der erste Halteabschnitt (4.2) und der Roboter mit dem Endeffektor (5.1) innerhalb des Hohlraums (3) untergebracht sind.

2. Ein System nach Anspruch 1, wobei die Verbindung zwischen dem Roboterpositionierer (4) und dem Roboter (5) ferner einen Zwischenhalteabschnitt (4.3) umfasst, durch den der Roboterpositionierer (4) konfiguriert ist, um mit dem Roboter (5) gekoppelt zu werden, wobei sich dieser Zwischenhalteabschnitt (4.3) von einem ersten Ende (4.3.1), das mit dem zweiten Ende (4.2.2) des ersten Halteabschnitts (4.2) verbunden ist, und einem zweiten Ende (4.3.2) gegenüber dem ersten Ende (4.3.1) erstreckt, wobei die Verbindung zwischen dem Zwischenhalteabschnitt (4.3) und dem ersten Halteabschnitt (4.2) die Verbindung ist, die einen Freiheitsgrad aufweist, der einer Drehung um die vertikale Achse (V) entspricht.

3. Ein System nach Anspruch 2, wobei der Roboterpositionierer (4) eine Vielzahl von Halteabschnitten umfasst, die zwischen ihnen angelenkt sind und eine kinematische Kette bilden, die sich vom ersten Halteabschnitt (4.2) und dem Zwischenhalteabschnitt (4.3) erstreckt.

4. Ein System nach Anspruch 3, wobei die Vielzahl von Halteabschnitten einen zweiten Halteabschnitt (4.4) umfasst, der sich von einem ersten Ende (4.4.1), das mit dem zweiten Ende (4.2.2) des ersten Halteabschnitts (4.2) verbunden ist, und einem zweiten Ende (4.4.2) erstreckt, das mit dem ersten Ende (4.3.1) des Zwischenhalteabschnitts (4.3) verbunden ist, und wobei in der Roboterlagerposition die Vielzahl von Halteabschnitten ebenfalls innerhalb des Hohlraums (3) untergebracht sind.

5. Ein System nach einem der vorhergehenden Ansprüche, wobei der vertikale Halter (4.1) ein oberes Ende (4.1.1) umfasst, das einen zweiten Freiheitsgrad aufweist, der einer vertikalen Verschiebung gemäß einer Richtung parallel zur vertikalen Achse (V) entspricht, die mindestens zwischen einer ersten unteren Position und einer zweiten oberen Position verschiebbar ist, wobei sich das erste Ende (4.2.1) des ersten Halteabschnitts (4.2) am oberen Ende (4.1.1) des vertikalen Halters (4.1) befindet; und wobei sich in der Roboterlagerposition das obere Ende (4.1.1) des vertikalen Halters (4.1) in der ersten unteren Position befindet.

6. Ein System nach einem der vorhergehenden Ansprüche, wobei die polygonale Basis viereckig ist.

7. Ein System nach einem der vorhergehenden Ansprüche, wobei der vertikale Halter (4.1) entlang des ersten vertikalen Eckpunkts (1.1) als ein Vorsprung aus dem prismatischen Körper (2) angeordnet ist.

8. Ein System nach einem der vorhergehenden Ansprüche, wobei die Haltestruktur (1) ferner Verschiebungsmittel (6) umfasst, die in der Basis des prismatischen Körpers (2) angeordnet sind.

9. Ein System nach einem der vorhergehenden Ansprüche, wobei die Haltestruktur (1) einen polygonalen Basishalter (7) umfasst, auf dem der prismatische Körper (2) auf eine feste Weise gehalten wird, und wobei gemäß einer Draufsicht orthogonal zur vertikalen Achse (V) der polygonale Basishalter (7) über den prismatischen Körper (2) hinausgeht und die Basis des prismatischen Körpers (2) teilweise umgibt, so dass diese Basis des prismatischen Körpers (2) in den Basishalter (7) eingebettet ist.

10. Ein System nach Anspruch 9, wobei der polygonale Basishalter (7) einen vorstehenden Abschnitt (7.1) umfasst, der von jedem Eckpunkt des polygonalen Basishalters (7) aus demselben vorsteht, und das Verschiebungsmittel (6) ein Rad umfasst, das an jedem vorstehenden Abschnitt (7.1) angeordnet ist.

11. Ein System nach einem der vorhergehenden Ansprüche, das mindestens eine erste Roboterarbeitsposition aufweist, wobei der vertikale Halter (4.1) um die vertikale Achse (V) relativ zu seiner Position in der Roboterlagerposition des Systems gedreht wird, so dass sich die kinematische Kette, die mindestens durch den ersten Halteabschnitt (4.2) und den Roboter (5) mit dem Endeffektor (5.1) gebildet wird, vom Inneren des Hohlraums (3) wegbewegt und außerhalb dieses Hohlraums (3) bleibt.

12. Ein System nach Anspruch 5 und einem der vorhergehenden Ansprüche, das mindestens eine zweite Roboterarbeitsposition aufweist, wobei der vertikale Halter (4.1) um die vertikale Achse (V) und relativ zu seiner Position in der Roboterlagerposition des Systems gedreht und von der ersten unteren Position in die zweite obere Position verschoben wird, so dass sich die kinematische Kette, die mindestens durch den ersten Halteabschnitt (4.2) und den Roboter (5) mit dem Endeffektor (5.1) gebildet wird, von der Haltestruktur (1) wegbewegt.

13. Ein System nach Anspruch 2 und einem der vorhergehenden Ansprüche, wobei der Zwischenhalteabschnitt (4.3) des Positionierers (4) mit einem ersten Abschnitt (5.2) des Roboters (5) verbunden ist, so dass der erste Abschnitt (5.2) des Roboters (5) einen einzigen Halteabschnitt mit dem Zwischenhalteabschnitt (4.3) für den Positionierer (4) bildet.

14. Ein System nach einem der vorhergehenden Ansprüche, wobei sich die Öffnung des Hohlraums (3) zusätzlich horizontal entlang einer zweiten Seitenfläche (2.2) neben der ersten Seitenfläche (2.1) erstreckt, wobei sich die zweite Seitenfläche (2.2) mindestens teilweise zwischen dem zweiten vertikalen Eckpunkt (1.2) und einem dritten vertikalen Eckpunkt (1.3) erstreckt.

15. Ein System nach einem der vorhergehenden Ansprüche, wobei in der Roboterlagerposition der erste Halteabschnitt (4.2) zu einer zweiten Seitenfläche (2.2) hin ausgerichtet ist.

16. Ein System nach einem der Ansprüche 1-14, wobei in der Roboterlagerposition der erste Halteabschnitt (4.2) zu einer dritten Seitenfläche (2.3) neben einer zweiten Seitenfläche (2.2) hin ausgerichtet ist.

17. Ein System nach einem der vorhergehenden Ansprüche, das ferner einen Roboter (5) umfasst, der mit dem Roboterpositionierer (4) am zweiten Ende (4.2.2) des ersten Halteabschnitts (4.2) gekoppelt ist, wobei in der Roboterlagerposition der Roboter (5) innerhalb des Hohlraums (3) untergebracht ist.

## Revendications

1. Un système de robot d'assistance chirurgicale pour positionner un robot chirurgical (5), le système comprenant une structure de support (1) et un positionneur de robot (4), dans lequel :
la structure de support (1) est configurée pour supporter le positionneur de robot (4) et comprend :
- un corps prismatique (2) avec une base polygonale et avec des sommets verticaux (1.1, 1.2, 1.3, 1.4) perpendiculaires à la base délimitant les faces latérales (2.1, 2.2, 2.3, 2.4) du corps prismatique (2), et
- une cavité (3) avec une ouverture qui part au moins d'un premier sommet vertical (1.1) et s'étend horizontalement le long d'une première face latérale (2.1) jusqu'à
un deuxième sommet vertical (1.2) adjacent au premier sommet vertical (1.1) le positionneur de robot (4) est configuré pour positionner le robot (5) et comprend :
- un support vertical (4.1) disposé le long du premier sommet vertical (1.1) et comprend un premier degré de liberté correspondant à une rotation par rapport à un axe vertical (V) coïncidant avec le premier sommet vertical (1.1) de la structure de support (1) ; et
- au moins une première section de support (4.2) s'étendant horizontalement depuis une première extrémité (4.2.1) située au niveau du support vertical (4.1) et une deuxième extrémité (4.2.2) opposée à la première extrémité (4.2.1) ;
dans lequel
le positionneur de robot (4) est configuré pour être couplé, au niveau de la deuxième extrémité (4.2.2) de la première section de support (4.2), au robot (5), le robot (5) comprenant un effecteur terminal (5.1) ; et
le système comporte au moins une position de rangement du robot dans laquelle la première section de support (4.2) et le robot avec l'effecteur terminal (5.1) sont logés à l'intérieur de la cavité (3).

2. Un système selon la revendication 1, dans lequel la liaison entre le positionneur de robot (4) et le robot (5) comprend en outre une section de support intermédiaire (4.3) par l'intermédiaire de laquelle le positionneur de robot (4) est configuré pour être couplé au robot (5), cette section de support intermédiaire (4.3) s'étendant depuis une première extrémité (4.3.1) reliée à la deuxième extrémité (4.2.2) de la première section de support (4.2) et une deuxième extrémité (4.3.2) opposée à la première extrémité (4.3.1), la liaison entre la section de support intermédiaire (4.3) et la première section de support (4.2) étant la liaison ayant un degré de liberté correspondant à une rotation autour de l'axe vertical (V).

3. Un système selon la revendication 2, dans lequel le positionneur de robot (4) comprend une pluralité de sections de support articulées entre elles formant une chaîne cinématique qui s'étend depuis la première section de support (4.2) et la section de support intermédiaire (4.3).

4. Un système selon la revendication 3, dans lequel la pluralité de sections de support comprend une deuxième section de support (4.4) s'étendant depuis une première extrémité (4.4.1) reliée à la deuxième extrémité (4.2.2) de la première section de support (4.2) et une deuxième extrémité (4.4.2) reliée à la première extrémité (4.3.1) de la section de support intermédiaire (4.3), et dans lequel, à la position de rangement du robot, la pluralité de sections de support sont également logées à l'intérieur de la cavité (3).

5. Un système selon l'une quelconque des revendications précédentes, dans lequel le support vertical (4.1) comprend une extrémité supérieure (4.1.1) ayant un deuxième degré de liberté correspondant à un déplacement vertical selon une direction parallèle à l'axe vertical (V), pouvant se déplacer au moins entre une première position inférieure et une deuxième position supérieure, la première extrémité (4.2.1) de la première section de support (4.2) étant située à l'extrémité supérieure (4.1.1) du support vertical (4.1) ; et dans lequel, dans la position de rangement du robot, l'extrémité supérieure (4.1.1) du support vertical (4.1) se trouve dans la première position inférieure.

6. Un système selon l'une quelconque des revendications précédentes, dans lequel la base polygonale est quadrangulaire.

7. Un système selon l'une quelconque des revendications précédentes, dans lequel le support vertical (4.1) est disposé le long du premier sommet vertical (1.1) sous la forme d'une saillie hors du corps prismatique (2).

8. Un système selon l'une quelconque des revendications précédentes, dans lequel la structure de support (1) comprend en outre des moyens de déplacement (6) disposés dans la base du corps prismatique (2).

9. Un système selon l'une quelconque des revendications précédentes, dans lequel la structure de support (1) comprend un support de base polygonal (7) sur lequel le corps prismatique (2) est supporté de manière fixe, et dans lequel, selon une vue en plan orthogonale à l'axe vertical (V), le support de base polygonal (7) s'étend au-delà du corps prismatique (2) et entoure partiellement la base du corps prismatique (2) de sorte que cette base du corps prismatique (2) est encastrée dans le support de base (7).

10. Un système selon la revendication 9, dans lequel le support de base polygonal (7) comprend une partie saillante (7.1) faisant saillie à partir de chaque sommet du support de base polygonal (7) hors de celui-ci, et le moyen de déplacement (6) comprend une roue disposée au niveau de chaque partie saillante (7.1).

11. Un système selon l'une quelconque des revendications précédentes comporte au moins une première position de travail du robot dans laquelle le support vertical (4.1) est tourné autour de l'axe vertical (V) par rapport à sa position dans la position de rangement du robot du système, de sorte que la chaîne cinématique formée au moins par la première section de support (4.2) et le robot (5) avec l'effecteur terminal (5.1) s'éloigne de l'intérieur de la cavité (3) et reste à l'extérieur de cette cavité (3).

12. Un système selon la revendication 5 et l'une quelconque des revendications précédentes comporte au moins une deuxième position de travail du robot dans laquelle le support vertical (4.1) est pivoté autour de l'axe vertical (V) et par rapport à sa position dans la position de rangement du robot du système, et déplacé de la première position inférieure vers la deuxième position supérieure, de sorte que la chaîne cinématique formée au moins par la première section de support (4.2) et le robot (5) avec l'effecteur terminal (5.1) s'éloigne de la structure de support (1).

13. Un système selon la revendication 2 et l'une quelconque des revendications précédentes, dans lequel la section de support intermédiaire (4.3) du positionneur (4) est reliée à une première section (5.2) du robot (5) de telle sorte que la première section (5.2) du robot (5) constitue une seule section de support avec la section de support intermédiaire (4.3) pour le positionneur (4).

14. Un système selon l'une quelconque des revendications précédentes, dans lequel l'ouverture de la cavité (3) s'étend en outre horizontalement le long d'une deuxième face latérale (2.2) adjacente à la première face latérale (2.1), la deuxième face latérale (2.2) s'étendant au moins partiellement entre le deuxième sommet vertical (1.2) et un troisième sommet vertical (1.3).

15. Un système selon l'une quelconque des revendications précédentes, dans lequel, dans la position de rangement du robot, la première section de support (4.2) est orientée vers une deuxième face latérale (2.2).

16. Un système selon l'une quelconque des revendications 1 à 14, dans lequel, dans la position de rangement du robot, la première section de support (4.2) est orientée vers une troisième face latérale (2.3) adjacente à une deuxième face latérale (2.2).

17. Un système selon l'une quelconque des revendications précédentes, comprenant en outre un robot (5) couplé au positionneur de robot (4) à la deuxième extrémité (4.2.2) de la première section de support (4.2), dans lequel, dans la position de rangement du robot, le robot (5) est logé à l'intérieur de la cavité (3).
